# EUROPEAN PATENT SPECIFICATION

(11) **EP 2 501 394 B1**
(45) Date of publication and mention of the grant of the patent: **29.07.2015**
(21) Application number: 10782138.1
(22) Date of filing: 17.11.2010
(51) Int. Cl.: A61K 35/54, A23L 1/275, A61P 19/02

(54) **EGGSHELL MEMBRANE FORMULATIONS FOR USE IN ALLEVIATING JOINT PAIN**
EIERSCHALENMEMBRANFORMULIERUNGEN ZUR VERWENDUNG BEI DER LINDERUNG VON GELENKSCHMERZEN
FORMULATIONS DE MEMBRANE DE COQUILLE D'OEUF POUR UTILISATION DANS LE SOULAGEMENT DES DOULEURS ARTICULAIRES

(30) Priority: 17.11.2009 US 261921 P; 16.11.2010 US 946924
(43) Date of publication of application: 26.09.2012
(73) Proprietor: U.S. Nutraceuticals LLC dba Valensa International, Eustis, FL 32726 (US)
(72) Inventor: MINATELLI, John, Eustis Florida 32726 (US); HILL, Stephen, Ocala Florida 34471 (US); MICHAEL,Cielensky, Florida 33327 (US)
(74) Representative: Ferreccio, Rinaldo
(86) International application number: PCT/US2010/056969
(87) International publication number: WO 2011/062953

(56) References cited:
- WO-A2-02/102394
- US-A1- 2004 180 025
- US-A1- 2006 159 816
- US-A1- 2009 170 808
- US-B1- 6 258 855
- KEVIN J RUFF ET AL: "Eggshell membrane in the treatment of pain and stiffness from osteoarthritis of the knee: a randomized, multicenter, double-blind, placebo-controlled clinical study" CLINICAL RHEUMATOLOGY ; JOURNAL OF THE INTERNATIONAL LEAGUE OF ASSOCIATIONS FOR RHEUMATOLOGY, vol. 28, no. 8, 2 April 2009 (2009-04-02), pages 907-914, XP019724858 SPRINGER-VERLAG, LO ISSN: 1434-9949 DOI: 10.1007/S10067-009-1173-4
- RUFF K J ET AL: "Eggshell membrane: a possible new natural therapeutic for joint and connective tissue disorders. Results from two open-label human clinical studies" CLINICAL INTERVENTIONS IN AGING, vol. 4, 9 June 2009 (2009-06-09), pages 235-240, XP002621029
- Anonymous: "Biova-BiovaFlex(TM)"[Online] 27 October 2009 (2009-10-27), XP002620900 Natural Product Insider Retrieved from the Internet: URL:http://www.naturalproductinsider.com/n ews/2009/10/biova-biovaflex.aspx> [retrieved on 2011-02-07]

## Description

### Field of the Invention

This invention relates to eggshell membrane applications, and more particularly, this invention relates to treating and alleviating symptoms of joint pain using eggshell membranes.

### Background of the Invention

It is known to use eggshell membrane compositions to alleviate everyday joint aches and pains, reduce joint discomfort, and improve joint mobility, flexibility and function. These eggshell membrane preparations are used to promote a normal inflammatory response and improve motion and provide some antioxidant activity.

U.S. Patent Publication Nos. 2008/0234195 and 2009/0104173 and U.S. Patent No. 6,946,551 disclose various compositions derived from eggshell membrane that include hyaluronic acid in combination with naturally occurring constituents derived from eggshell membrane that can be used in one aspect for treating joint pain and joint problems. There are also compounds such as BiovaFlex™ as a natural joint health ingredient, which includes proteins and peptides derived from hydrolyzed, water-soluble egg membrane used for joint treatment.

### Summary of the Invention

The present invention is defined by the claims.

A composition comprising a fowl eggshell membrane and hyaluronic acid having a mol. weight between 0.5 and 100 KDa and as taxanthin for use according to the claims is disclosed. A method to treat and alleviate symptoms of joint pain in an individual is accomplished by administering a therapeutic amount of fowl eggshell membrane in synergistic combination with other active constituents in an oral dosage form. A composition is also disclosed.

### Detailed Description

A method to treat and alleviate symptoms of joint pain in an individual is disclosed and includes administering a therapeutic amount of fowl eggshell membrane in synergistic combination with other active constituents in an oral dosage form. The eggshell membrane preparation in one example has been processed to increase water solubility or enriched to increased concentration of active substances. In another example, the formulation delivers 0.5-1000 mg of eggshell membrane daily dose. The preferred formulation delivers 40-500 mg eggshell membrane per daily dose.

In yet another aspect, the formulation is supplemented with various molecular weight polymers of hyaluronic acid or sodium hyaluronate (hyaluronan) derived from microbial fermentation, eggshell or animal tissue whether in pure or crude form. For example, the formulation delivers 50-1000 mg hyaluronan per daily dose. The hyaluronic acid is derived from a biofermentation process and has a molecular weight between 0.5 and 100 kilodaltons (kDa).

Astaxanthin is added in another aspect and has synergistic effects in combination with the other ingredients. The formulation is supplemented to deliver 0.1-12 mg astaxanthin per daily dose in one example. The astaxanthin is derived from *Haematococcus pluvialis* algae, *Pfaffia,* krill, or by synthetic routes, in the free diol, monoester or diester form in another example.

A natural or synthetic cyclooxygenase-1 or -2 inhibitor such as aspirin, acetaminophen, steroids, prednisone, NSAIDs, turmeric, Curcumin, boswellia and the like is added in another example. The composition also includes a gamma-linoleic acid rich oil such as from Borage (*Borago officina*/*is* L.), Safflower (*Carthamus tinctorius* L.) and the like, and is combined with an n-3 (omega-3) fatty acid rich oil such as from: fish oil, algae oil, flax seed oil, soybean oil, perilla seed oil, chia seed oil and the like - with the n-3 fatty acid being alpha-linolenic, stearidonic, eicosapentaenoic or docosapentaenoic acid. The composition is supplemented with collagen in any of its various forms in one example.

In another example, the composition is combined with anti-inflammatory and/or joint health promoting compounds such as preparations of: green lipped mussel (*Perna canaliculus*), *Boswellia serrata,* turmeric (*Curcuma longa*), stinging nettle (*Urtica* dioica), Andrographis, Cat's claw (*Uncaria tomentosa),* white willow *(Salix alba),* bromelain, Vitamin D, Magnesium, milk protein concentrates, fatty acid esters, methylsulfonylmethane (MSM), chondroitin sulfate, glucosamine sulfate, glucosamine hydrochloride, s-adenosyl methionine, proanthocyanidins, procyanidins or flavonoids. The ingredients are formulated in a chewable dosage form in another example, which could include a tablet or "gummy."

In yet another example, a pharmaceutically acceptable formulation of eggshell membrane is combined or supplemented with at least one of the following: glucosamine sulfate, chondroitin sulfate, collagen, astaxanthin, hyaluronic acid, methylsulfonylmethane, a gamma-linoleic acid or omega-3 fatty acid rich oil or cyclooxygenase inhibitor for the treatment of symptoms related to joint diseases such as, but not limited to osteoarthritis and rheumatoid arthritis.

In still another example, a dietary supplement acceptable formulation of eggshell membrane is combined or supplemented with at least one of the following: glucosamine sulfate, chondroitin sulfate, collagen, astaxanthin, hyaluronic acid, methylsulfonylmethane, a gamma-linoleic acid or omega-3 fatty acid rich oil or cyclooxygenase inhibitor for the treatment of symptoms related to joint diseases such as, but not limited to osteoarthritis and rheumatoid arthritis.

In another example, a medical food acceptable formulation of eggshell membrane is combined or supplemented with at least one of the following: glucosamine sulfate, chondroitin sulfate, collagen, astaxanthin, methylsulfonylmethane, a gamma-linoleic acid or omega-3 fatty acid rich oil or cyclooxygenase inhibitor for the treatment of symptoms related to joint diseases such as, but not limited to osteoarthritis and rheumatoid arthritis.

As to astaxanthin, it is beneficial and synergistic in combination with the other components, and as noted in the commonly assigned U.S. Patent Publication No. 2008/0124391, astaxanthin (3,3'-dihydroxy-β, β-carotene-4, 4'dione) CAS [471-53-4], is a keto carotenoid pigment naturally accumulated via the diet in marine animals such as salmon, shrimp, red seabream and lobster and in birds such as flamingoes. Astaxanthin also occurs in certain microalgae such as *Haematococcus pluvialis* and in yeasts such as *Phaffia* species. The highest concentration, up to four percent of dry matter, occurs in *Haematococcus.* It can be chemically synthesized and obtained in naturally occurring stereoisomer form.

Astaxanthin, although related to other carotenoids such as beta-carotene, zeaxanthin and lutein, is a more powerful antioxidant. Astaxanthin is particularly potent in quenching singlet oxygen and has over five hundred times the ability to quench singlet oxygen as alpha-tocopherol. This antioxidant activity of astaxanthin is thought to be responsible for the wide range of health-promoting properties it exhibits, including skin and eye protection from damage by UV-light, anti-inflammatory activity, modulation or promotion of the immune response, reduction in aging processes and benefits to heart, liver, joints and prostate.

Astaxanthin is a pigment imparting the pinkish-red hue to the flesh of salmon and trout, and the coloring in the carapaces of shrimp, lobsters and crayfish. The astaxanthin molecule has two asymmetric carbons located at the 3 and 3' positions of the cyclohexenone rings on either end of the molecule. Different enantiomers of the molecule result from how the hydroxyl groups (--OH) are attached to the carbon atoms at these centers of asymmetry. The three possible enantiomers of astaxanthin are (3R,3'R), (3S,3'S) and (3R,3'S; meso).

Free astaxanthin and its mono- and diesters from *Haematococcus* have optically pure (3S,3'S)-chirality. The (3S,3'S) isomer of astaxanthin is found in the skin and flesh of some salmonid fish.

Natural astaxanthin extract is an oily, viscous dark red lipophilic extract. The extract contains free astaxanthin, astaxanthin fatty acid mono-esters and astaxanthin fatty acid di-esters along with triglycerides and other lipophilic compounds. Carotenoid pigments from different sources of *Haematococcus pluvialis* include in some examples astaxanthin (total) 81-99% (which comprises free astaxanthin 1-5%; astaxanthin monoesters 46-79%; astaxanthin diesters 10-39%); β-carotene 0-5%; lutein 1-11%; canthaxanthin 0-5.5%; and other carotenoids 1-9%.

Naturally derived astaxanthin is typically the 3S,3'S stereoisomer found in *Haematococcus* algae or 3R,3R', primarily found in *Phaffia* yeast. Synthetic astaxanthin has a more complex stereoisomeric profile due to the non-stereo selectivity from the reaction conditions used in its manufacture. *Haematococcus pluvialis* contains mono and diesterified astaxanthin as the predominant forms of astaxanthin, while *Phaffia* and synthetically produced astaxanthin lack these esterifications.

Natural astaxanthin extracts contain astaxanthin in E and Z isomeric configurations.

Natural astaxanthin extract derived from *Haematococcus pluvialis* as an example includes astaxanthin stereoisomers as follows: (3S,3'S) 100%; (3S,3'R) and (3R,3'S) 0%; (3R,3'R) 0%, with the geometric isomer proportions, expressed as a percentage of the total astaxanthin, of about: E-astaxanthin 59%; 9Z-astaxanthin 15%; 13Z-astxanthin 26%, and non-astaxanthin carotenoid levels of about: 0.3% β-carotene. 0.07% lutein, 0.3% canthaxanthin and 1.3% total other carotenoids.

It includes low molecular weight hyaluronic acid (HA) that is a major hydrodynamic component of synovial fluid and is a natural absorbent and lubricant for bones. It is excellent for bioavailability and maximizes interaction with target synovial cells and has anti-inflammatory mechanisms and down regulates pro-inflammatory mediators and neuro-peptide production and improves the symptoms of osteoarthritis. The HA's with molecular weights within the range of 0.5 to about 1 times 10⁶ DA were generally more effective in reducing indices of synovial inflammation and storing rheological properties of SF (visco-induction) than HA's with molecular weight greater than 2.3 times 10⁶ DA.

In induced uveitis, astaxanthin showed dose dependent ocular anti-inflammatory activity by suppression of NO, PGE-2 and TNF-Alpha by directly blocking NO synthase activity. It reduces C-Reactive Protein (CRP) blood levels: In human subjects with high risk levels of CRP three months of astaxanthin treatment resulted in 43% of patients serum CRP levels to drop below the risk level. Astaxanthin is so powerful it completely negates the pro-oxidant activity of Vioxx, which is known to cause cellular membrane lipid peroxidation leading to heart attack and stroke. Astaxanthin is absorbed by in vitro lens epithelial cells where it dramatically suppresses UVB induced lipid peroxidative mediated cell damage at umol/L concentrations suggesting use for the prevention of cataracts. In human trials astaxanthin at 4 mgs/day completely prevented joint pain following strenuous knee exercise when compared to untreated subjects.

## Claims

1. A composition comprising a therapeutically effective amount of a fowl eggshell membrane in combination with hyaluronic acid having a molecular weight between 0.5 and 100 kilodaltons (kDa) and astaxanthin in an oral dosage form for use in the treatment of joint pain in an individual.

2. The composition for use according to Claim 1, wherein said eggshell membrane comprises an eggshell membrane preparation that has been enriched to increased concentration of active substances.

3. The composition for use according to Claim 1, wherein the composition delivers 0.5-1000 mg of fowl eggshell membrane per daily dose.

4. The composition for use according to Claim 1, wherein the composition delivers 40-500 mg of fowl eggshell membrane per daily dose.

5. The composition for use according to Claim 1, wherein the hyaluronic acid is derived from microbial fermentation, eggshell or animal tissue in pure or crude form.

6. The composition for use according to Claim 1, wherein the composition delivers 0.1-12 mg astaxanthin per daily dose.

7. The composition for use according to Claim 6, wherein the astaxanthin is derived from *Haematococcus pluvialis* algae, *Pfaffia,* krill, or by synthetic routes, in the free diol, monoester or diester form.

8. The composition for use according to Claim 1, and further comprising a natural or synthetic cyclooxygenase-1 or -2 inhibitor including at least one of aspirin, acetaminophen, steroids, prednisone, and NSAIDs.

9. The composition for use according to Claim 1, and further comprising an n-3 (omega-3) fatty acid rich oil derived from fish oil, algae oil, flax seed oil, soybean oil, perilla seed oil, and chia seed oil, wherein the n-3 fatty acid comprises at least one of alpha-linolenic, stearidonic, eicosapentaenoic or docosapentaenoic acid.

10. The composition for use according to Claim 1, and further comprising at least one of an anti-inflammatory and joint health promoting compound derived from at least one of green lipped mussel (*Pema canaliculus*), *Boswellia serrata,* turmeric (*Curcuma longa*), stinging nettle (*Urtica dioica*)*,* Andrographis, Cat's claw *(Uncaria tomentosa),* white willow *(Salix alba),* bromelain, Vitamin D, Magnesium, milk protein concentrates, fatty acid esters, methylsulfonylmethane (MSM), chondroitin sulfate, glucosamine sulfate, glucosamine hydrochloride, and s-adenosyl methionine.

11. A dietary supplement composition consisting of 100-500 mg of hydrolyzed fowl eggshell membrane preparation, 10-40 mg sodium hyaluronate (< 100 .kDa), 0.5-12 mg Astaxanthin, *Boswellia serrata* preparation providing at least 30-100 mg Boswellic acids.

## Patentansprüche

1. Zusammensetzung umfassend eine therapeutisch wirksame Menge einer Geflügeleierschalmembran in Kombination mit Hyaluronsäure mit einem Molekulargewicht zwischen 0,5 und 100 Kilodalton (kDa) und Astaxanthin in oraler Darreichungsform zur Verwendung bei der Behandlung von Gelenkschmerz bei einer Person.

2. Zusammensetzung zur Verwendung nach Anspruch 1, wobei die Eierschalmembran ein Eierschalmembranpräparat umfasst, das auf eine erhöhte Konzentration an Wirkstoffen angereichert wurde.

3. Zusammensetzung zur Verwendung nach Anspruch 1, wobei die Zusammensetzung 0,5-1000 mg Geflügeleierschalmembran pro Tagesdosis freisetzt.

4. Zusammensetzung zur Verwendung nach Anspruch 1, wobei die Zusammensetzung 40-500 mg Geflügeleierschalmembran pro Tagesdosis freisetzt.

5. Zusammensetzung zur Verwendung nach Anspruch 1, wobei die Hyaluronsäure aus mikrobieller Fermentation, der Eierschale oder dem Tiergewebe in reiner oder unbehandelter Form stammt.

6. Zusammensetzung zur Verwendung nach Anspruch 1, wobei die Zusammensetzung 0,1-12 mg Astaxanthin pro Tagesdosis freisetzt.

7. Zusammensetzung zur Verwendung nach Anspruch 6, wobei das Astaxanthin aus *Haematococcus pluvialis* algae, *Pfaffia,* Krill stammt oder auf synthetischen Wegen in den freien Diol-, Monoester- oder Diesterformen abgeleitet ist.

8. Zusammensetzung zur Verwendung nach Anspruch 1, und weiter umfassend einen natürlichen oder synthetischen Cyclooxygenase-1- oder -2-Hemmer, der mindestens eines der Folgenden aufweist: Aspirin, Acetaminophen, Steroide, Prednison und NSAIDs.

9. Zusammensetzung zur Verwendung nach Anspruch 1, und weiter umfassend ein n-3 (Omega-3)-Fettsäure reiches Öl, das aus Fischöl, Algenöl, Flachssamenöl, Sojaöl, Perillasamenöl und Chiasamenöl stammt, wobei die n-3-Fettsäure mindestens eines der Folgenden umfasst: alpha-Linolen-, Stearidon-, Eicosapentaen- oder Docosapentaensäure.

10. Zusammensetzung zur Verwendung nach Anspruch 1, und weiter umfassend mindestens eines der Folgenden: eine entzündungshemmende und gelenkgesundheitsfördernde Verbindung, die aus mindestens einem der Folgenden stammt: Grünlippmuschel (*Perna canaliculus*), *Baswellia serrata,* Kurkuma (*Curcuma longa*), Brennnessel (*Urtica dioica*)*,* Andrographis, Katzenklaue (*Uncaria tomentosa),* Silberweide *(Salix alba),* Bromelain, Vitamin D, Magnesium, Milchproteinkonzentrate, Fettsäureester, Methylsulfonylmethan (MSM), Chondroitinsulfat, Glucasaminsulfat, Glucosaminhydrochlorid und s-Adenosylmethionin.

11. Nahrungsergänzungsmittelzusammensetzung bestehend aus 100-500 mg eines hydrolysierten Eierschalmembranpräparats; 10-40 g Natriumhyaluronat (< 100 kDa); 0,5-12 mg Astaxanthin; *Boswellia serrata* Präparat, das mindestens 30-100 mg Boswelliasäuren bereitstellt.

## Revendications

1. Composition comprenant une quantité thérapeutiquement efficace d'une membrane de coquille d'oeuf de volaille en combinaison avec de l'acide hyaluronique ayant un poids moléculaire compris entre 0,5 et 100 kilodaltons (kDa) et de l'astaxanthine dans une forme pharmaceutique orale pour utilisation dans le traitement de la douleur articulaire chez un individu.

2. Composition pour utilisation selon la revendication 1, **caractérisée en ce que** ladite membrane de coquille d'oeuf comprend une préparation de membrane de coquille d'oeuf qui a été enrichie à une concentration augmentée de substances actives.

3. Composition pour utilisation selon la revendication 1, **caractérisée en ce que** la composition délivre 0,5 à 1000 mg de membrane de coquille d'oeuf de volaille par dose journalière.

4. Composition pour utilisation selon la revendication 1, **caractérisée en ce que** la composition délivre 40 à 500 mg de membrane de coquille d'oeuf de volaille par dose journalière.

5. Composition pour utilisation selon la revendication 1, **caractérisée en ce que** l'acide hyaluronique est dérivé de fermentation microbienne, de coquille d'oeuf ou de tissu animal sous forme pure ou brute.

6. Composition pour utilisation selon la revendication 1, **caractérisée en ce que** la composition délivre 0,1 à 12 mg d'astaxanthine par dose journalière.

7. Composition pour utilisation selon la revendication 6, **caractérisée en ce que** l'astaxanthine est dérivée d'algues *Haematococcus pluvialis, Pfaffia,* krill, ou par des voies synthétiques, sous forme de diol fibre, monoester ou diester.

8. Composition pour utilisation selon la revendication 1, comprenant en outre un inhibiteur de cyclooxygénase 1 ou 2 naturel ou synthétique comprenant au moins l'un parmi l'aspirine, l'acétaminophène, des stéroïdes, la prednisolone, et des AINS.

9. Composition pour utilisation selon la revendication 1, comprenant en outre une huile riche en acide gras n-3 (oméga-3) dérivée d'huile de poisson, d'huile d'algue, d'huile de lin, d'huile de soja, d'huile de graines de périlla, et d'huile de graines de chia, dans laquelle l'acide gras n-3 comprend au moins l'un de l'acide alpha-linolénique, stéaridonique, eicosapentaénoïque ou docosapentaénoique.

10. Composition pour utilisation selon la revendication 1, comprenant en outre au moins l'un d'un anti-inflammatoire et d'un composé améliorant la santé des articulations dérivé d'au moins l'un parmi les moules vertes (*Perna canaliculus*), *Boswellia serrata,* le curcuma (Curcuma longa), l'ortie (Urtica dioica), l'andrographis, la griffe de chat *(Uncaria tomentosa*), le saule blanc *(Salix alba),* la broméline, la vitamine D, le magnésium, des concentrés de protéines du lait, des esters d'acides gras, la méthylsulfonylméthane (HSH), le sulfate de chondroïtine, le sulfate de glucosamine, le chlorhydrate de glucosamine, et la S-adénosyl-méthionine.

11. Composition de suppléments alimentaires constitués de 100 à 500 mg de préparation de membrane coquille d'oeuf de volaille hydrolisée, 10 à 40 mg de hyaluronate de sodium (< 100 kDa), 0,5 à 12 mg d'astaxanthine, une préparation de *Boswellia serrata* fournissant au moins 30 à 100 mg d'acides boswelliques.
